# EUROPEAN PATENT APPLICATION

(11) **EP 2 955 177 A2**
(43) Date of publication of application: **16.12.2015**
(21) Application number: 14749194.8
(22) Date of filing: 04.02.2014
(51) Int. Cl.: C07D 403/04, C07C 303/32, A61K 31/4178, A61P 31/12

(54) **ALKYL [(S)-1-((S)-2-{5-[4-(4-{2-[(S)-1-((S)-2-METHOXYCARBONYLAMINO-3-METHYL-BUTYRYL)-PYRROLIDINE-2-YL]-3H-IMIDAZOLE-4-YL}-BUTA-1,3-DIENYL)-PHENYL]-1H-IMIDAZOLE-2-YL}-PYRROLIDINE-1-CARBONYL)-2-METHYL-PROPYL]-CARBAMATE NAPHTHALENE-1,5-DISULFONATE, PHARMACEUTICAL COMPOSITION, MEDICINAL AGENT AND METHOD FOR TREATMENT OF VIRAL DISEASES**

(30) Priority: 07.02.2013 RU 2013105163
(71) Applicant: Ivachtchenko, Alexandre Vasilievich, Moskovskaya obl. 141700 (RU); Ivashchenko, Andrey Alexandrovich, Moscow 127576 (RU); Savchuk, Nikolay Filippovich, California 92067 (US)
(72) Inventor: IVACHTCHENKO, Alexandre Vasilievich, Dolgoprudnyj Moskovskaya obl., 141700 (RU)
(74) Representative: Henrion, Oliver
(86) International application number: PCT/RU2014/000079
(87) International publication number: WO 2014/123456

(57) **Abstract**

The present invention relates to NS5A inhibitors, to novel pharmaceutical composition, antiviral medicament, method for prophylaxis and treatment of viral diseases, in particular caused by hepatitis C (HCV) virus and hepatitis GBV-C virus.

Alkyl [(S)-1-((S)-2-{5-[4-(4-{2-[(S)-1-((S)-2-methoxycarbonylamino-3-methylbutyryl)-pyrrolidin-2-yl]-3H-imidazol-4-yl}-buta-1,3-diynyl)-phenyl]-1H-imidazol-2-yl}-pyrrolidine-1-carbonyl)-2-methyl-propyl]-carbamate naphthaline-1,5-disulfonate of the general formula 1, possibly in crystalline or polycrystalline form, have been proposed, 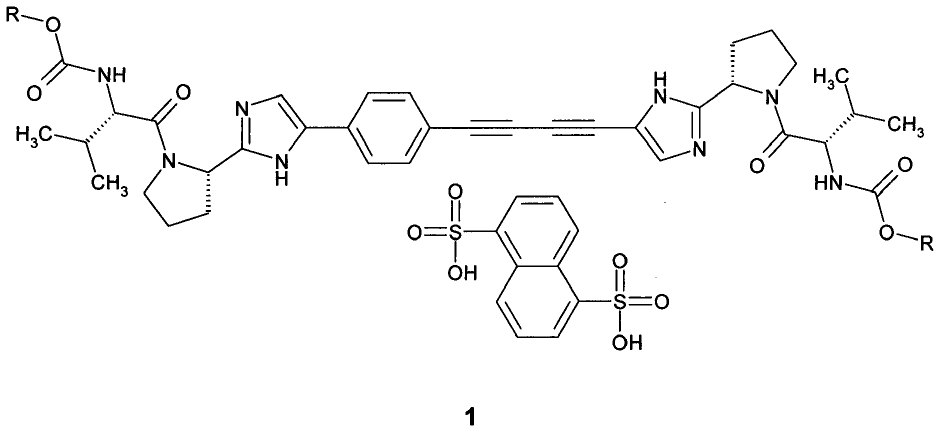 wherein: R is C₁-C₃ alkyl.

## Description

### Field of the invention

The present invention relates to NS5A inhibitors, to novel pharmaceutical composition, to antiviral medicament, to a method for prophylaxis and treatment of viral infections, particularly caused by hepatitis C viruses (HCV).

### Background of the invention

Virus infections may cause a great number of diseases that creates a hazard to human health and existence of mankind. For the last 20 years no less than 30 essentially new infectious agents have been discovered, among them: HIV, viral hepatitis, acute and long-lasting diarrhea, hemorrhagic fever (Ebola, Venezuelan, Brazilian, Rift valleys) [a) Lednicky J.A., Rayner J.O. Uncommon respiratory pathogens. Curr. Opin. Pulm. Med. 2006, 12(3), 235-239. b) Hayden F.G. Respiratory viral threats. Curr. Opin. Infect. Dis. 2006, 19(2), 169-178]. In particular, special alarm is about the risk of infection with so named avian influenza. [a) Liu J.P. Avian influenza-a pandemic waiting to happen, J. Microbiol. Immunol. Infect. 2006, 39(1), 4-10. b) Henter J.I.; Chow C.B.; Leung C.W, Lau Y.L. Cytotoxic therapy for severe avian influenza A (H5N1) infection. Lancet. 2006 367(9513), 870-873. Review]. In accordance with statistics, 60-65% of epidemic infections are of viral etiology. Because of the complexity of interaction in triad "virus - host's organism - drug", most of current antiviral drugs induce side effects in the course of therapy and form resistant virus strains [Jain R., Clark N.M., Diaz-Linares M., Grim S.A. Limitations of current antiretroviral agents and opportunities for development. Curr. Pharm. Des. 2006, 12(9), 1065-1074.]. At present, the number of antiviral drugs that could be used in clinical experience is extremely limited - only 43 low molecular weight substances [http://integrity.prous.com/integrity] that is far from satisfying requirements of prophylaxis and treatment of viral diseases. Moreover, there is a significant number of viral infections causing diseases for treatment of which there are no chemotherapeutic remedies. It concerns, for example, to the diseases caused by viruses of papilloma, adenoviruses, herpes-6, variola, syndrome SARS, hemorrhagic fevers, fever of the Western Nile, avian influenza and so on [De Clercq E. Recent highlights in the development of new antiviral drugs. Curr Opin Microbiol. 2005, 8(5), 552-560].

Hepatitis C virus falls into the category of Flaviviruses (genus Flaviviridae), together with the other important human pathogens, such as yellow fever virus, West Nile virus, Dengue virus and hepatitis GBV-C virus. Flaviviruses exhibit similar genome structures, among other things they have the gene encoding the nonstructural NS5A protein. NS5A protein plays an important role in the replication of the viral RNA genome, being a structural component of the viral replication complex. As far as this protein has been validated now in clinical trials as a target for design of medicaments for treating long-lasting hepatitis C, NS5A is considered to be a promising target for other listed above clinically important Flaviviruses as well.

Thus, the development of novel antiflavivirus medicaments, especially possessing high activity and low toxicity is of great importance now.

There are some publications in patent literature, dedicated to various derivatives of 2-pyrrolidin-2-yl-1H-imidazoles, which are ligands of non-structural NS5A protein and suppress hepatitis C virus (HCV) [WO 2008021927A2, WO 2009020825A1, WO 2009020828A1, WO 2010065668A1, WO2010065681A1, WO2010096302A1, WO2010096462A1, WO2010096777A1, WO2010111534A1, WO2010111673A1, WO2010117635A1, WO2010117977A1], among other, the patent is known RU 2452735 (2012) RF where methyl [(S)-1-((S)-2-{5-[4-(4-{2-[(S)-1-((S)-2-methoxycarbonylamino-3-methyl-butyryl)-pyrrolidin-2-yl]-3H-imdazol-4-yl}-buta-1,3-diynyl)-phenyl]-1H-imidazol-2-yl}-pyrrolidine-1-carbonyl)-2-methyl-propyl]-carbamate of formula **A** and its dihydrochloride **A·2HCl** have been described.

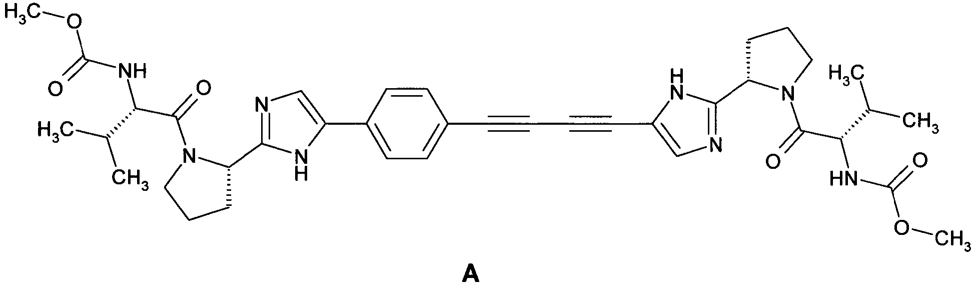

However, because of the cumbersomeness of molecules purification of these compounds is very complicated.

So, searching for novel highly effective antiflavivirus medicaments now is still one of the principal directions in the developing of new pharmacological agents for treating vide and diversified types of viral infections.

### Disclosure of the invention

In the context of this invention, terms are generally defined as follows:
**"Alkyl"** means an aliphatic hydrocarbon straight or branched chain with 1-12 carbon atoms. Branched means the alkyl chain with at least one or more "lower alkyl" substituents. Alkyl group may have one or more substituents of the same or different structure ("alkyl substituent") including halogen, alkenyloxy, cycloalkyl, aryl, heteroaryl, heterocyclyl, aroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonylheteroaralkyloxy, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, Rₖ^{a}Rₖ₊₁^{a}NC(=S)-, Rₖ^{a}Rₖ₊₁^{a}NSO₂-, where Rₖ^{a} and Rₖ₊₁^{a} independently of each other represent "amino group substituents", the meanings of which are defined in this section, for example, hydrogen, alkyl, aryl, aralkyl, heteroaralkyl, heterocyclyl or heteroaryl, or Rₖ^{a} and Rₖ₊₁^{a} together with the N-atom, they are attached to, form through Rₖ^{a} and Rₖ₊₁^{a} 4-7-membered heterocyclyl or heterocyclenyl. The preferred alkyl groups are methyl, trifluoromethyl, cyclopropylmethyl, cyclopentylmethyl, ethyl, n-propyl, *iso*-propyl, n-butyl, *tert-butyl,* n-pentyl, 3-pentyl, methoxyethyl, carboxymethyl, methoxycarbonylmethyl, ethoxycarbonylmethyl, benzyloxycarbonylmethyl and pyridylmethyloxycarbonylmethyl. The preferred "alkyl substituents" are cycloalkyl, aryl, heteroaryl, heterocyclyl, hydroxy, alkoxy, alkoxycarbonyl, aralkoxy, aryloxy, alkylthio, heteroarylthio, aralkylthio, alkylsulfonyl, arylsulfonyl, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, annelated arylheterocyclenyl and annelated arylheterocyclyl.
**"Amino group substituent"** means a substituent attached to amino group. Amino group substituent represents hydrogen, alkyl, cycloalkyl, aryl, heteroaryl, heterocyclyl, acyl, aroyl, alkylsulfonyl, arylsulfonyl, heteroarylsulfonyl, alkylaminocarbonyl, arylaminocarbonyl, heteroarylaminocarbonyl, heterocyclylaminocarbonyl, alkylaminothiocarbonyl, arylaminothiocarbonyl, heteroarylaminothiocarbonyl, heterocyclylaminothiocarbonyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonylalkyl, aralkoxycarbonylalkyl, heteroaralkyloxycarbonylalkyl.
**"Active component"** (drug-substance) means a physiologically active compound of synthetic or other (biotechnological, vegetable, animal, microbe and so on) origins exhibiting pharmacological activity which is an active ingredient of pharmaceutical composition employing in production and preparation of medicaments.
**"Medicament" -** is a compound (or a mixture of compounds as a pharmaceutical composition) in the form of tablets, capsules, injections, ointments and other ready forms intended for restoration, improvement or modification of physiological functions in humans and animals, and for treatment and prophylaxis of diseases, for diagnostics, anesthesia, contraception, cosmetology and others.
**"Therapeutic kit"** represents a simultaneously administered combination of two or more medicaments exhibiting different mechanism of pharmacological action and directed to various biotargets taking part in disease process.
**"Pharmaceutical composition"** means a composition comprising a compound of the general formula I and at least one of the component selected from the group consisting of pharmaceutically acceptable and pharmacologically compatible fillers, solvents, diluents, carriers, auxiliary, distributing and sensing agents, delivery agents, such as preservatives, stabilizers, filler, disintegrators, moisteners, emulsifiers, suspending agents, thickeners, sweeteners, flavouring agents, aromatizing agents, antibacterial agents, fungicides, lubricants, and prolonged delivery controllers, the choice and suitable proportions of which depend on the type and way of administration and dosage. Examples of suitable suspending agents are ethoxylated isostearyl alcohol, polyoxyethene, sorbitol and sorbitol ether, microcrystalline cellulose, aluminum metahydroxide, bentonite, agar-agar and tragacant and their mixtures as well. Protection against action of microorganisms can be provided by various antibacterial and antifungal agents, such as, for example, parabens, chlorobutanole, sorbic acid, and similar compounds. Composition may also contain isotonic agents, such as, for example, sugar, sodium chloride, and similar compounds. Prolonged action of composition may be achieved by agents slowing down absorption of active ingredient, for example, aluminum monostearate and gelatine. Examples of suitable carriers, solvents, diluents and delivery agents include water, ethanol, polyalcohols and their mixtures, natural oils (such as olive oil) and organic esters (such as ethyl oleate) for injections. Examples of fillers are lactose, milk-sugar, sodium citrate, calcium carbonate, calcium phosphate and the like. Examples of disintegrators and distributors are starch, alginic acid and its salts, and silicates. Examples of suitable lubricants are magnesium stearate, sodium lauryl sulfate, talc and polyethylene glycol of high molecular weight. Pharmaceutical composition for peroral, sublingval, transdermal, intramuscular, intravenous, subcutaneous, local or rectal administration of active ingredient, alone or in combination with another active compound may be administered to human and animals in a standard administration form, or in a mixture with traditional pharmaceutical carriers. Suitable standard administration forms include peroral forms such as tablets, gelatin capsules, pills, powders, granules, chewing-gums and peroral solutions or suspensions; sublingval and transbuccal administration forms; aerosols; implants; local, transdermal, subcutaneous, intramuscular, intravenous, intranasal or intraocular forms and rectal administration forms.

The authors have discovered unknown before stereoisomeric alkyl [(S)-1-((S)-2-{5-[4-(4-{2-[(S)-1-((S)-2-methoxycarbonylamino-3-methyl-butyryl)-pyrrolidin-2-yl]-3H-imidazol-4-yl}-buta-1,3-diynyl)-phenyl]-1H-imidazol-2-yl}-pyrrolidine-1-carbonyl)-2-methyl-propyl]-carbamate naphthaline-1,5-disulfonate of the general formula 1, where:

R represents C₁-C₃ alkyl.

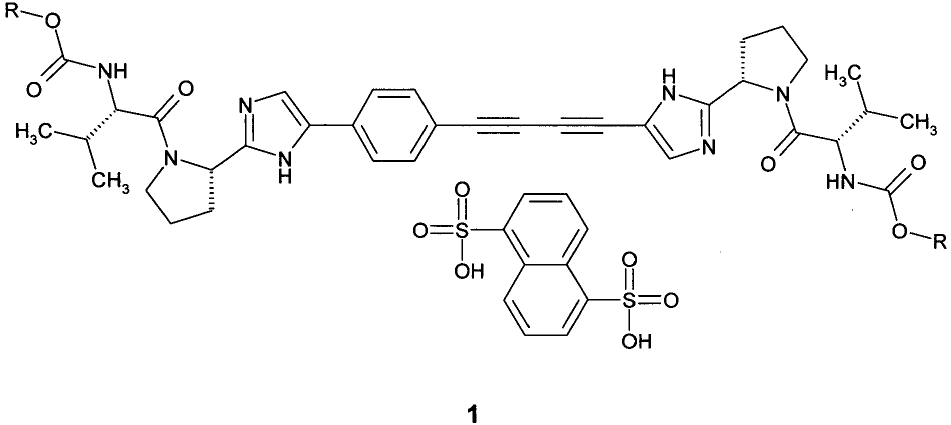

According to the present invention, the more preferable is also methyl [(S)-1-((S)-2-{5-[4-(4-{2-[(S)-1-((S)-2-methoxycarbonylamino-3-methyl-butyryl)-pyrrolidin-2-yl]-3H-imidazol-4-yl}-buta-1,3-diynyl)-phenyl]-1H-imidazol-2-yl}-pyrrolidine-1-carbonyl)-2-methylpropyl]-carbamate naphthalene-1,5-disulfonate 1.1

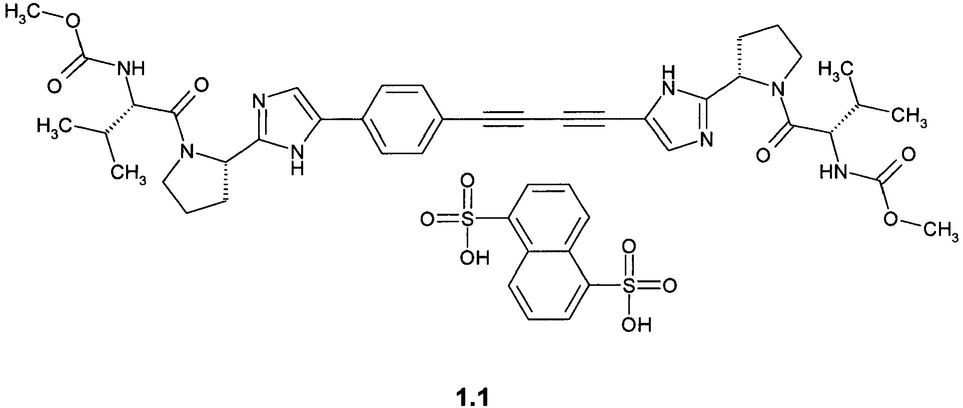

The base of the general formula 1 was prepared using the procedure described in patent RU 2452735 RF (2012) p. 66-68.

According to the present invention naphthalene-1,5-disulfonate salts of the general formula 1 are prepared by interaction of bases of formula 1 with naphthalene-1,5-disulfonic acid in methanol, ethanol or *iso*-propanol.

The salts of the general formula 1 prepared according to the present invention are distinguished by high purity required for use in pharmacopoeia, and they could be well stored at reduced temperatures (4.7 ° C).

The structure of the novel compounds and their purity were confirmed by analytical chromatography and LCMS. Compounds were named using ChemDraw program (Chembridge Soft Inc.).

The subject of the present invention is protein NS5A inhibitor, representing a compound of the general formula 1 or formula 1.1, possibly in crystalline or polycrystalline form.

The subject of the present invention is a pharmaceutical composition for the treatment and prophylaxis of diseases caused by the hepatitis C virus and hepatitis GBV-C virus, comprising therapeutically effective amount of compounds of the general formula 1 or formula 1.1 as protein NS5A inhibitor.

Pharmaceutical compositions may include pharmaceutically acceptable excipients. Pharmaceutically acceptable excipients mean diluents, auxiliary agents and/or carriers applied in the sphere of pharmaceutics. According to the present invention a pharmaceutical composition together with compound of the general formula 1 or its crystalline form, or polycrystalline form may include other active components provided that they do not give rise to undesirable effects, such as allergic reaction.

When needed to use the pharmaceutical compositions according to the present invention in clinical practice they may be mixed for preparation of various forms, at this they may include traditional pharmaceutical carriers; for example, peroral forms (such as tablets, gelatinous capsules, pills, solutions, or suspensions); forms for injections (such as solutions or suspensions for injections, or dry powder for injections, which needs only addition of injectable water before usage); local forms (such as ointment or solutions).

According to the present invention the carriers used in pharmaceutical compositions represent carriers which are used in the sphere of pharmaceutics for preparation of commonly used forms, among them binding agents, greasing agents, disintegrators, solvents, diluents, stabilizers, suspending agents, colorless agents, taste flavors are used in peroral forms; antiseptic agents, solubilizers, stabilizers are used in forms for injections; base materials, diluents, greasing agents, antiseptic agents are used in local forms.

Pharmaceutical composition was prepared by mixing of at least one compound of the general formula **1** or its crystalline form or polycrystalline form with inert filler and/or solvent.

Clinical dose of pharmaceutical composition comprising a compound of the general formula **1** as an active component at patients can be corrected depending on: therapeutic efficiency and bio-accessibility of active ingredients in patients' organism, rate of their exchange and removal from organism, age, gender, and severity of patient's symptoms. Thus, the daily intake for adults normally is 10∼500 mg. While preparing pharmaceutical compositions according to the present invention as a dose unit the above effective dose is to be taken into consideration, at this, each dose unit of composition should contain 10∼500 mg of compound of formula **1.** Following the instructions of physician or pharmacist, the medicaments may be taken several times over specified periods of time (preferably, from one to six times).

The subject of the present invention is a medicament in the form of tablets, capsules or injections placed in pharmaceutically acceptable packing for prophylaxis and treatment of diseases caused by hepatitis C virus and hepatitis GBV-C virus, comprising as protein NS5A inhibitor at least one compound of the general formula **1** or formula **1.1,** or novel pharmaceutical composition in therapeutically effective amount.

The subject of the present invention is a therapeutic kit for prophylaxis and treatment of diseases caused by hepatitis C virus and hepatitis GBV-C virus, comprising at least one compound of the general formula **1** or formula **1.1,** or novel pharmaceutical composition, or novel medicament in therapeutically effective amount.

Therapeutic kits for prophylaxis and treatment of the above flavivirus diseases, including hepatitis C and hepatitis GBV-C, along with the medicaments disclosed in the present invention, may include: inhibitors inosine-5-monophosphate dehydrogenase, for example, Ribavirin (allowed) and Ribamidine; inhibitors of NS3 hepatisis C protease, for example, Telaprevir, Siluprevir and SCH-503034; inhibitors of RNK-polimeraze NS5B, for example, XTL-2125; alpha-glucosidase inhibitors, for example, aminocarbohydrate Selgozivir; and also TLR-receptor agonists, hepatoprotectors, cyclosporines, various proteins (for example, interferons), antibodies, vaccines etc.

For combination therapy any classes of agents that may be useful when combined with bis-azoles of the present invention and which may mean, for example, nucleoside and non-nucleoside inhibitors of the HCV polymerase, protease inhibitors, helicase inhibitors, NS4B inhibitors and medicinal agents that functionally inhibit the internal ribosomal entry site (IRES) and other medicaments that inhibit HCV cell attachment or virus entry, HCV RNA translocation, transcription, replication or HCV maturation, concentration or virus release. Specific compounds in these classes and useful in this invention include, but are not limited to, macrocyclic, heterocyclic and linear HCV protease inhibitors such as telaprevir (VX-950), boceprevir (SCH-503034), narlaprevir (SCH-900518), ITMN-191 (R-7227), TMC-435350 (a.k.a. TMC-435), MK-7009, BI-201335, BI-2061 (ciluprevir), BMS-650032, ACH-1625, ACH-1095 (HCV NS4A protease co-factor inhibitor) VX-500, VX-813, PHX-1766, PHX2054, IDX-136, IDX-316, ABT-450 EP-013420 (and congeners) and VBY-376; the nucleosidic HCV polymerase (replicase) inhibitors useful in the invention include, but are not limited to, R7128, PSI-7851, IDX-184, IDX-102, R1479, UNX-08189, PSI-6130, PSI-938 and PSI-879 and various other nucleoside and nucleotide analogs and HCV inhibitors including (but not limited to) those derived as 2'-C-methyl modified nucleoside and nucleotide; and 7'-deaza modified nucleoside and nucleotide. Non-nuclosidic HCV polymerase (replicase) inhibitors useful in the invention, include, but are not limited to, HCV-796, HCV-371, VCH-759, VCH-916, VCH-222, ANA-598, MK-3281, ABT-333, ABT-072, PF-00868554, BI-207127, GS-9190, A-837093, JKT-109, GL-59728 and GL-60667.

In addition, NS5A inhibitors of the present invention may be used in combination with cyclophyllin and immunophyllin antagonists (for example, without limitation, DEBIO compounds, NM-811, as well as cyclosporine and its derivatives), kinase inhibitors, inhibitors of heat shock proteins (for example, HSP90, HSP70), other immunomodulatory agents that may include, without limitation, interferons (alpha-, beta-, omega-, gamma-, lambda or synthetic), such as Intron A™, Roferon- A™, Canferon-A300™, Advaferon™, Infergen™, Humoferon™, Sumiferon MP™, Alfaferon™, IFN-p™, Feron ™, and the like, polyethylene glycol derivatized (pegylated) interferon compounds, such as: PEG interferon-α-2a (Pegasys™), PEG interferon-α-2b (PEGIntron™), pegylated IFN-α-con 1 and the like; long acting formulations and derivatives of interferon compounds, such as albumin-fused interferon, Albuferon™, Locteron™, and the like; interferons with various types of controlled delivery systems (e.g. ITCA-638, omega-interferon delivered by the DUROS subcutaneous delivery system); compounds that stimulate the synthesis of interferon in cells, such as resiquimod and the like; interleukins; compounds that enhance the development of type 1 helper T cell response, such as SCV-07 and the like; TOLL-like receptor agonists, such as: CpG-10101 (action), isotorabine, ANA773 and the like; thymosin α-1, ANA-245 and ANA-246, histamine dihydrochloride, propagermanium; tetrachlorodecaoxide; ampligen; IMP-321; KRN-7000; antibodies, such as: civacir, XTL-6865 and the like and prophylactic and therapeutic vaccines, such as: Inno Vac, HCV E1 E2/MF59 and the like. In addition, any of the above-described methods involving administration of an NS5A inhibitor, a Type 1 interferon receptor agonist (e.g., an IFN-α) and a Type n interferon receptor agonist (e.g., IFN-γ) can be augmented by administration of an effective amount of TNF-α antagonist. Exemplary, non-limiting TNF-α antagonists that are suitable for use in such combination therapies include ENBREL™ and HUMIRA™.

In addition, NS5A inhibitors of the present invention may be used in combination with antiprotozoans and other antivirals thought to be effective in the treatment of HCV infection, such as, the prodrug nitazoxanide. Nitazoxanide can be used as an agent in combination with the compounds disclosed in this invention as well as in combination with other agents useful in treating HCV infection such as peginterferon alfa-2a and ribavarin (see, for example, Rossignol, JF and Keeffe, EB, Future Microbiol. 3:539-545, 2008).

NS5A inhibitors of the present invention may also be used in combination with alternative forms of interferons and pegylated interferons, ribavirin or its analogs (e.g., Tarabavarin, levovirion), microRNA, small interfering RNA compounds (e.g., SIRPLEX-140-N) and the like, nucleotide or nucleoside analogs, immonoglobulins, hepatoprotectants, anti-inflammatory agents and other inhibitiors of NS5A. Inhibitors of other targets in the HCV life cycle include NS3 helicase inhibitors; NS4A co-factor inhibitors, antisense oligonucleotide inhibitors, such as ISIS-14803, AVI-4065 and the like; vector-encoded short hairpin RNA (shRNA); HCV specific ribozymes such as heptazyme, RPI, 139199 and the like; entry inhibitors such as HepeX-C, HuMax-HepC and the like; alpha glucosidase inhibitors such as celgosivir, UT-231 B and the like; KPE-02003002 and BIVN 401 and IMPDH inhibitors. Other illustrative compounds HCV inhibitor compounds include those disclosed in the known scientific and patent publications.

Additionally, combinations of, for example, ribavirin and interferon may be administered as combination therapy with, at least one compound of formula **1** or compound **1.1.** The present invention is not limited to the aforementioned classes or compounds and contemplates known and new compounds and combinations of biologically active agents. It is intended that combination therapies of the present invention include any chemically compatible combination of bis-azoles of this inventive group with other compounds of the inventive group or other compounds outside of the inventive group, as long as the combination does not eliminate the antiviral activity of the compound of this inventive group or the antiviral activity of the pharmaceutical composition itself.

Combination therapy can be sequential, that is treatment with one agent first and then a second agent (for example, where each stage of the treatment comprises a different compound of the present invention or where one stage of the treatment comprises a compound of the present invention and the other comprises one or more biologically active agents) or it can be treatment with both agents at the same time. Sequential therapy can include a reasonable time after the completion of the first therapy before beginning the second therapy. Treatment with both agents at the same time can be in the same daily dose or in separate doses. Combination therapy need not be limited to two agents and may include three or more agents. The dosages for both concurrent and sequential combination therapy will depend on absorption, distribution, metabolism and excretion rates of the components of the combination therapy as well as other factors known to one of skill in the art. Dosage values will also vary with the severity of the condition to be alleviated. For any particular subject specific dosage regimens and schedules may be adjusted over time according to the individual's need and the professional judgement of the person administering or superivising the administration of the combination therapy.

The subject of the present invention is a method for prophylaxis and treatment of diseases caused by hepatitis C virus and hepatitis GBV-C virus consisting in introduction of therapeutically effective amount of at least one compound of the general formula **1** or formula **1.1,** or novel pharmaceutical composition, or novel medicament, or therapeutic kit.

Below the invention is described by means of specific examples which illustrate, but not limit the scope of the invention.

### Example 1.

The general method for preparation of naphthalene-1,5-disulfonates of the general formula **1.** Base of the general formula **1** (5 g) was dissolved in methanol (150 ml). The obtained solution was filtered through Celite, and a solution of tetrahydrate of naphthalene-1,5-disulfonic acid (1.05 eq.) in methanol was added to it at stirring in amount of 50 ml on 5 g of the base. The mixture was stirred for 45-60 min (but not more, because increasing the time of stirring resulted in developing extremely finely divided precipitate), precipitated solid was collected by centrifugation, washed two times with methanol, ether, (after each washing the solid was centrifugated), transferred with ether into a round-bottom flask and dried on a rotator evaporator, and then subjected to lyophilization. It gave compound of the general formula 1, yield above 75% naphthalene-1,5-disulfonate:
**1.1,** LCMS, 711 (M+¹H) ¹H NMR (DMSO-D6, 400 MHz) δ 0.96-1.05 (d, J₁ = 6.1, 12H), 1.70 (m, 2H), 1.79 (m, 2H), 1.94 (m, 2H), 2.04 (m, 2H), 2.31 (m, 2H), 3.48 (m, 2H), 3.56 (s, 6H), 3.58 (m, 2H), 4.16 (m, 2H), 4.56 (m, 2H), 6.85 (d, J₁ = 8.0, 2H), 6.98 (s, 1 H), 7.20 (bd, 2H), 7.58 (d, J₁ = 7.8, 2H), 7.64 (s, 1 H), 7.83 (bs, 2H), 8.02 (t, J₁ = 8.0, 2H), 8.29 (d, J₁ = 7.8, 2H), 9.56 (d, J₁ = 8.0, 2H), 11.60 (bs, 2H), purity of compounds was 98.8% according to data of HPLC method;
**1.2,** LCMS, 739 (M+¹H) ¹H NMR (DMSO-D6, 400 MHz) δ 0.96-1.05 (d, J₁ = 6.1, 12H), 1.28 (t, J₁ = 7.1, 6H), 1.70 (m, 2H), 1.79 (m, 2H), 1.94 (m, 2H), 2.04 (m, 2H), 2.31 (m, 2H), 3.48 (m, 2H), 3.58 (m, 2H), 4.16 (m, 2H), 4.19 (q, J₁ = 7.1, 4H), 4.56 (m, 2H), 6.85 (d, J₁ = 8.0, 2H), 6.98 (s, 1 H), 7.20 (bd, 2H), 7.58 (d, J₁ = 7.8, 2H), 7.64 (s, 1 H), 7.83 (bs, 2H), 8.02 (t, J₁ = 8.0, 2H), 8.29 (d, J₁ = 7.8, 2H), 9.56 (d, J₁ = 8.0, 2H), 11.60 (bs, 2H), purity of compounds was 98.6% according to data of HPLC method;
**1.3,** LCMS, 767 (M+¹H), ¹H NMR (DMSO-D6, 400 MHz) δ 0.96-1.05 (d, J₁ = 6.1, 12H), 1.03 (t, J₁ = 7.1, 6H), 1.60 (m, 4H), 1.70 (m, 2H), 1.79 (m, 2H), 1.94 (m, 2H), 2.04 (m, 2H), 2.31 (m, 2H), 3.48 (m, 2H), 3.58 (m, 2H), 3.96 (t, J₁ = 7.1, 4H), 4.16 (m, 2H), 4.56 (m, 2H), 6.85 (d, J₁ = 8.0, 2H), 6.98 (s, 1 H), 7.20 (bd, 2H), 7.58 (d, J₁ = 7.8, 2H), 7.64 (s, 1 H), 7.83 (bs, 2H), 8.02 (t, J₁ = 8.0, 2H), 8.29 (d, J₁ = 7.8, 2H), 9.56 (d, J₁ = 8.0, 2H), 11.60 (bs, 2H), purity of compounds was 98.5% according to data of HPLC method;

If the purity of the product was below 98.5%, additional purification was carried out by means of another precipitation of naphthalene-1,5-disulfonate. For this purpose salt **1.1, 1.2** or **1.3** was transferred into base for the second time. In order to do so, the salt was suspended in ethanol (100 ml), then 10% solution of sodium carbonate (100 ml) was added to it and after complete dissolution of the salt methylene chloride (400 ml) was added. Organic layer was separated, washed once with water and saturated solution of sodium chloride, dried over sodium sulfate, evaporated *in vacuo* at a temperature below 40°C. Then naphthalene-1,5-disulfonate of compounds **1.1, 1.2** or **1.3** was precipitated by the method described above.

For preparation of naphthalene-1,5-disulfonate with purity above 99% purification of base was carried out by method of column chromatography. Eluent - dioxane : toluene = 1:1.5. The middle fractions were combined, evaporated *in vacuo* at a temperature below 40°C. Then naphthalene-1,5-disulfonate of compounds **1.1, 1.2** or **1.3** was precipitated using the method mentioned above. Yield - 60%, purity - above 99%.

### Example 2.

### Determination of antiviral activity of the compounds of the general formula 1 (inhibiting ability of NS5A protein).

Inhibiting ability of compounds of formula **1** of NS5A protein was determined in the human hepatoma cell line Huh7, comprising subgenomic RNA-replicon HCV (genotype 1b, clon Con1). A version of immumoenzymatic assay (IEA) on viral protein NS5A in 96-well plate was used as an experimental method. Cytotoxicity of the compounds was estimated in parallel regime.

Cells Huh7 were seeded in 96-well plate (7.5x10³ cells to each well in 100 µl of culture medium). Solutions of the tested compounds in DMEM medium {DMEM 1X; Source: Cellgro; Catalogue: 10-013-CV} were prepared immediately before use. Eleven serial three fold dilutions with variation of concentrations from 20 nM to 0.2 pM were prepared. In 4 hours after seeding, serial dilutions of the compounds were added to the cells (100 µl to each well). Final concentration of tested compounds was varied from 10 nM to 0.1 pM, and DMSO - 0.5%. If it was necessary, higher concentrations of the disclosed azoles were investigated. Each dilution of the compound was tested on two identical wells. Then the cells were incubated for three days at 37°C/5% CO₂ and fixed by addition of acetone/methanol (1:1) mixture in amount of 250 µl/well. In 1 min the cells were washed 3 times with PBS (Phosphate Buffered Saline) solution. Then the cells were blocked by addition of 10% fetal calf serum in PBS solution in amount of 150 µl/well for 1 h at room temperature. After that the cells were incubated with mouse monoclonal antibodies to cor-antigen HCV, clon C7-50 (Source: Affinity BioReagents; Catalogue: MA1-080) (100 µl/well, working dilution - 1:500 in 10% fetal calf serum in PBS solution) for 2 h at 37°C. The cells were washed 6 times with PBS/0.05% Tween 20 solution, then, they were incubated for 1 h with goat anti-mouse immunoglobulin antibodies (conjugated with horseradish peroxidase, 100 µl/well, working dilution - 1:2500 in 10% fetal calf serum in PBS solution). The cells were washed 6 times with PBS/0.05% Tween 20 solution, once with PBS solution, after that substrate (1 tablet of o-phenylenediamine (oPD) + 12 ml citrate/phosphate buffer + 5 µl 30% H₂O₂) in amount of 100 µl/well was added. The plates were kept for 30 min in the dark at room temperature. The reaction was arrested by the addition of 2N H₂SO₄ in amount of 100 µl/well, and optical density (wavelength 490 nm) was measured by means of multiscan plate reader Victor3 V 1420 (Perkin Elmer). IC₅₀ values (azole concentration, lowering the level of virus RNA-replicon by 50%) for every tested bis-azole were calculated with the help of XLfit 4 program.

Cytotoxicity of the compounds of the general formula 1 was tested in experiments on the human hepatoma cell line Huh7. The amount of living cells was determined with the help of ATPLite kit (Perkin Elmer, Boston, USA) in accordance with manufacturer instructions. Cytotoxic action was estimated by seeding the cells into black microplate with transparent bottom (96 wells, 10⁴ cells to each well). Three independent repeats were used for each bis-azole. The tested azoles were added in 18 h, after that the cells were incubated together with the compounds for 96 h. Each well was washed two times with phosphate buffered saline (0.2 ml/well) and then the cells were lysed by addition of cell buffer (50 µl/well) (all mentioned reagents are included in ATPLite kit). The microplate was incubated for 5 min on a rotating platform at 600 r/min, after that 50 µl of substrate solution (a part of ATPLite kit) was added into each well. The microplate was incubated for additional 5 min on a rotating platform at 600 r/min, kept for 10 min in the dark; after that luminescence was measured using TopCount NXT instrument (Packard, Perkin Elmer). CC₅₀ Value corresponding to bis-azole concentration at which 50% of cells were ruined was used as quantitative characteristic for cytotoxicity estimation. Calculation of CC₅₀ value: for calculation of inhibition effectiveness (% Inh) the following equation was used: % Inh = [(L^{pos} - L^{ex})/ L^{pos} - L^{neg})] * 100%, where L^{pos} - positive control, luminescence in the wells with cells without compounds; L^{neg} - negative control, luminescence in the wells with medium without cells; L^{ex} - luminescence in wells with a compound of definite concentration.. Then, CC₅₀ values were calculated with the help of XLfit 4 program.

Test results for novel compounds of the general formula 1 give evidence of their high picomolar activity. Inhibitory activity of novel compounds towards genotype 1 b, 1 a and 2a HCV is represented in Table.

**Table. Inhibitory activity of naphthalene-1,5-disulfonates of the general formula 1 towards genotype 1 b, 1 a and 2a HCV**

| **Compound** | **gT1b** | **gT1a** | **gT2a** |
|---|---|---|---|
| | **FBS EC₅₀, nM** | | |
| **1.1** (R=CH₃) | < 1 nM | < 1 nM | < 1 nM |
| **1.2** (R=C₂H₅) | < 1 nM | < 1 nM | < 1 nM |
| **1.3** (R=C₃H₇) | < 1 nM | < 1 nM | < 1 nM |

### Example 3.

Investigation of storage of naphthalene-1,5-disulfonates of the general formula 1. All samples were stored in glass vials sealed with rubber cork and aluminum caps, in a refrigerator at a temperature of about ∼ 4.7 °C. The amount of admixtures was determined by method of internal normalization, detection was carried out using UV detector at 220 nm. Below are given the test results for compound 1.1 showing their stability at temperature 4.7 ° C.

| Storage conditions | Time of storage | | | | | | | |
|---|---|---|---|---|---|---|---|---|
| | Before the test | | 1 week | | 4 weeks | | 20 weeks | |
| | Max admixt ure, % | Sum of admixtures, % | Max admixture, % | Sum of admixtures, % | Max admixture, % | Sum of admixtures, % | Max admixture, % | Sum of admixtures, % |
| | Compound **1.1** | | | | | | | |
| Refrigerator, 4.7 °C | 0,342 | 1,312 | 0,348 | 1,369 | 0,341 | 1,506 | 0,298 | 1,493 |

### Example 4.

Preparation of a pharmaceutical composition in the form of tablets. Starch (1600 mg), grained lactose (1600 mg), talcum (400 mg) and compound **1.1** (1000 mg) were carefully mixed together. The prepared brick was crushed to granules and riddled through sieves, gathering granules of 14-16 mesh size. The obtained granules were pelletized into tablets of suitable form by 560 mg each.

**Example 5.** Preparation of pharmaceutical composition in the form of capsules. Compound **1.2** was carefully mixed with a powder of lactose in ratio 2:1. The resultant powdery mixture was packed by 300 mg into gelatinous capsules of suitable size.

### Example 6.

Preparation of pharmaceutical composition in the form of injectable compositions for intramuscular, intraperitoneal or subcutaneous injections. Compound 1.1(500 mg) was mixed with chlorobutanol (300 mg), propylene glycol (2 ml) and injectable water (100 ml). The resultant solution was filtered and placed into 1 ml ampoules which were sealed afterwards.

### Industrial applicability

The invention can be used in medicine and veterinary.

## Claims

1. Alkyl [(S)-1-((S)-2-{5-[4-(4-{2-[(S)-1-((S)-2-methoxycarbonylamino-3-methyl butyryl)-pyrrolidin-2-yl]-3H-imidazol-4-yl}-buta-1,3-diynyl)-phenyl]-1H-imidazol-2-yl}-pyrrolidine-1-carbonyl)-2-methyl-propyl]-carbamate naphthaline-1,5-disulfonate of the general formula **1,** possibly in crystalline or polycrystalline form 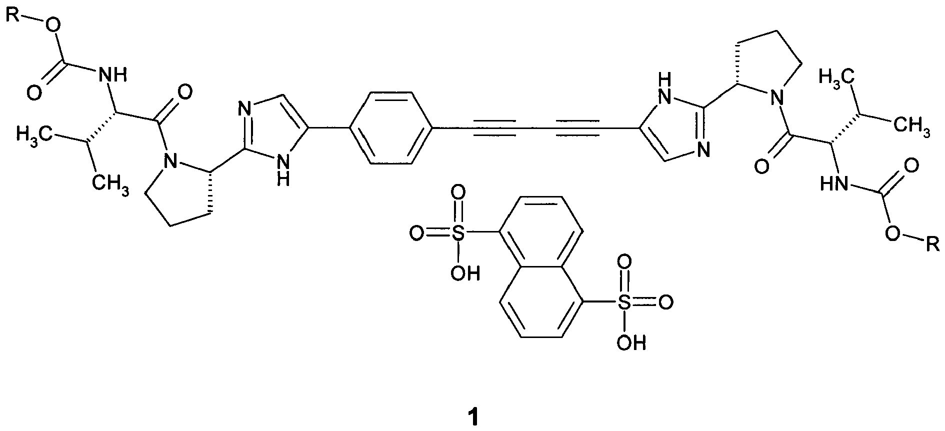 wherein: R is C₁-C₃ alkyl.

2. A compound according to claim 1 representing the compound of formula **1.1** possibly in crystalline or polycrystalline form.

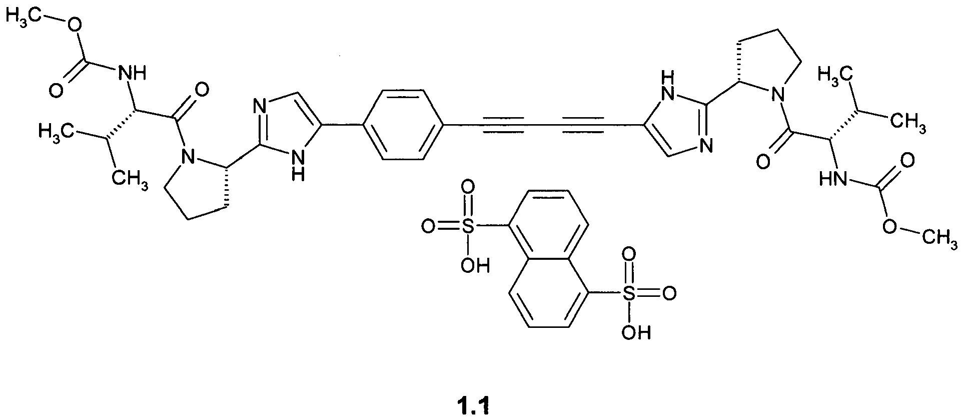

3. Alkyl [(S)-1-((S)-2-{5-[4-(4-{2-[(S)-1-((S)-2-methoxycarbonylamino-3-methylbutyryl)-pyrrolidin-2-yl]-3H-imidazol-4-yl}-buta-1,3-diynyl)-phenyl]-1H-imidazol-2-yl}-pyrrolidine-1-carbonyl)-2-methyl-propyl]-carbamate naphthaline-1,5-disulfonate, possibly in crystalline or polycrystalline form according to any of claims 1-2 exhibiting a property of protein NS5A inhibitor for pharmaceutical compositions or medicaments.

4. A pharmaceutical composition exhibiting a property of protein NS5A inhibitor for prophylaxis and treatment of diseases caused by hepatitis C virus and hepatitis GBC-C virus, comprising therapeutically effective amount of a compound according to claim 3 as an active component.

5. A medicament exhibiting a property of protein NS5A inhibitor for prophylaxis and treatment of diseases caused by hepatitis C virus and hepatitis GBC-C virus in the form of tablets, capsules or injections placed in pharmaceutically acceptable packing and comprising therapeutically effective amount of a compound according to claim 3 as an active component or pharmaceutical composition according to claim 4 in the therapeutically effective amount.

6. A therapeutic kit for prophylaxis and treatment of diseases caused by hepatitis C virus and hepatitis GBC-C virus comprising therapeutically effective amount of a compound according to any of claims 1-2, or pharmaceutical composition according to claim 4.

7. A method for prophylaxis and treatment of diseases caused by hepatitis C virus and hepatitis GBC-C virus consisting in introduction of therapeutically effective amount of a compound according to claim 3, or pharmaceutical composition according to claim 4, or medicament according to claim 5, or therapeutic kit according to claim 6.
